# EUROPEAN PATENT SPECIFICATION

(11) **EP 1 660 675 B1**
(45) Date of publication and mention of the grant of the patent: **31.12.2008**
(21) Application number: 04761572.9
(22) Date of filing: 20.07.2004
(51) Int. Cl.: C12Q 1/68

(54) **POLYMORPHISM OF THE IGF2 GENE AND IMPROVING PRODUCTION CHARACTERISTICS OF CATTLE**
POLYMORPHISMUS DES IGF2-GENS UND VERBESSERTE PRODUKTIONSEIGENSCHAFTEN VON RINDERN
POLYMORPHISME DE L'IGF2 ET AME LIORATION DES CARACTERISTIQUES DE PRODUCTION DES BOVINS

(30) Priority: 22.07.2003 US 489026 P
(43) Date of publication of application: 31.05.2006
(73) Proprietor: UNIVERSITY OF SASKATCHEWAN, Saskatoon, Saskatchewan S7N 4JB (CA)
(72) Inventor: Schmutz, Sheila Marie, Saskatoon, Saskatchewan S7K 3J5 (CA); Goodall, Julie Janine, Wilkie, Saskatchewan S0K 4W0 (CA)
(74) Representative: Gaunt, Robert John
(86) International application number: PCT/CA2004/001039
(87) International publication number: WO 2005/007881

## Description

### FIELD OF THE INVENTION:

The present invention relates generally to methods of selective breeding and management of cattle, and in particular to predicting production characteristics from a genotype.

### BACKGROUND:

Calf birth weight is considered one of the most critical factors in obtaining a live calf, and therefore is very important to the success of a cow-calf operation. Traditional belief among ranchers (Canadian Cattlemen magazine, Calving Special 2003) was that producers will have to sacrifice a high rate of gain in order to have a smaller calf at birth. Smaller birth weight much reduces the need for assistance at birthing and increases the survival rate of both the calves and dams. Parent animals who will produce offspring with birth weights that improve the chances of survival are thus often desirable. Birth weight is a highly heritable trait.

Weight gain by a livestock animal during its growth and development typically follows a tri-phasic pattern that is carefully managed by commercial producers and finishers. It is known that the efficiency of dietary caloric (feed) conversion to weight gain during an increment of time varies during these three phases, although little is known about the genetic or physiological basis of the variability.

The first phase of growth is considered to comprise that portion of a livestock animal's life from birth to weaning. Typically in commercial livestock feeding and finishing operations little attention is paid to this phase of growth, as food is provided by the mother and so there is little to be done in terms of food ration management.

A second growth phase comprises that portion of a livestock animal's life from weaning to attainment of musculo-skeletal maturity. As feed conversation efficiency is low during this phase, livestock producers usually restrict caloric intake in an effort to keep feed costs as low as practical without affecting the muscle maturation process. Limiting caloric intake does have the effect of prolonging this growth phase, but also typically results in animals with larger frame size, which is the primary aim of dietary management during this phase. During the second growth phase weight gain is associated with increases primarily in skeletal and muscle mass.

The third phase of growth is associated primarily with fat accumulation. During this phase, and after an animal has attained musculo-skeletal maturity, the efficiency of feed conversion is further reduced such that it requires even more feed to increase an animal's weight by a particular amount.

A further goal in breeding and herd management programs is to increase the proportion of high value cuts of meat in the finished animal, thereby increasing the value of the animal at slaughter. One measurement associated with higher value carcasses is rib eye area (REA) size. Since it is known that variability exists among animals with respect to feed conversion efficiency and the tendency to produce high value meat, producers have attempted to develop methods of screening animals in order to predict which ones will yield the greatest value as a finished product. One method of breeding and culling, based on parental animal frame size, is of some use in judging the potential of animals but does not realize the full genetic potential of an individual animal in terms of feed conversion efficiency and the proportion of high value meat cuts that it might eventually provide.

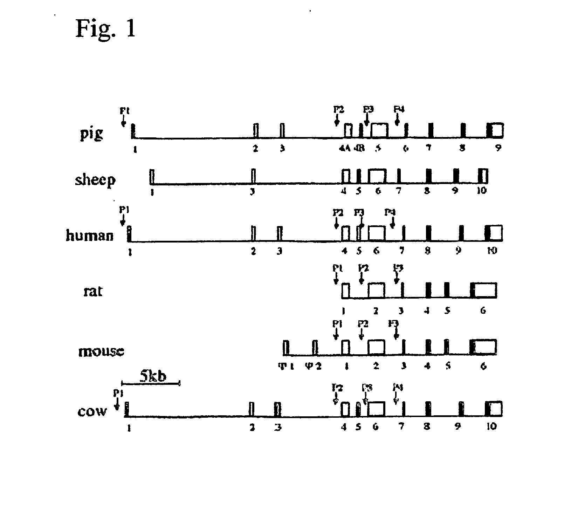

Insulin-like growth factor 2 (IGF2) is a 67 amino acid peptide hormone having multiple phenotypic effects on cellular growth and metabolism. The *IGF2* gene comprises 10 exons in pig (Amarger et al. 2002) and human (McLaren and Montgomery 1999), while the sheep *IGF2* gene has 9 exons (Fig. 1) (Ohlsen et al. 1994). No equivalent to human exon 2 has been found in sheep (Ohlsen et al. 1994). The mouse and rat *IGF2* have six exons and two pseudo or non-coding exons (Ohlsen et al. 1994) (Fig. 1).

IGF2 was previously thought to function primarily as a fetal and neonatal growth factor (De Chiara et al. 1990, 1991, Rotwein and Hall 1990, Giannoukakis et al. 1993). However, a role for *IGF2* during the postnatal development of lean meat carcass content of pigs (i.e. ham size) has recently been demonstrated (Jeon et al. 1999, Nezer et al. 1999, Amarger et al. 2002).

In several species such as mice, (De Chiara et al. 1991, Sasaki et al. 1992), rats (Ohlsson et al. 1993), humans (Kalscheuer et al. 1993, Giannoukakis et al. 1993), pigs (Nezer et al. 1999, Jeon et al. 1999), and sheep (McLaren and Montgomery 1999, Feil et al. 1998) the *IGF2* gene has been shown to be genomically imprinted. In these animals, the paternal allele is expressed throughout the development and lifetime of the animal.

### LITERATURE CITED (in Background):

Amarger V., M. Nguyen, A.S. Van Laere, M. Braunschweig, C. Nezer, M. Georges, and L. Andersson. 2002. Comparative sequence analysis of the INS-IGF2-H19 gene cluster in pigs. Mamm Genome 13(7):388-98.
Beever J.E., George P.D., Fernando R.L., Stormont C.J. and Lewin H.A. 1990. Associations between genetic markers and growth and carcass traits in a paternal halb-sib family of Angus cattle. J. Anim. Sci 68 : 337- 344
Canadian Cattleman's Association Calving Special. 2003.
De Chiara, T.M., Efstratiadis, A. and Robertson, E.J. 1990. A growth-deficiency phenotype in heterozygous mice carrying an insulin-like growth factor II gene disrupted by targeting. Nature 345: 78-82.
De Chiara, T.M., Robertson, E.J. and Efstratiadis, A. 1991. Parental imprinting of the mouse insulin-like growth factor two gene. Cell 64: 849-859.
Giannoukakis, N., Deal, C. Paquette, J. Goodyer, C.G. and Polychronakos, C. 1993. Paternal genomic imprinting of the human IGF2 gene. Nat. Genetics. 4: 98-101.
Goodall, J.J. 2002. Undergraduate thesis title: Characterization of the Insulin-like growth factor II gene in cattle.
Goodall, J.J. and Schmutz, S.M. 2203. Linkage Mapping of IGF2 on Cattle Chromosome 29. Anim. Genet. 34(4): 313.
Holtuizen P., Van der Lee F.M., Ikejiri K., Yamamoto M., and J.S. Sussenbach. 1990. Identification and initial characterization of a fourth leader exon and promoter of the human IGF2 gene. Biochim. Biophys. Acta 1087:341-3.
Jeon, J.T., Carlborg, O. Tornsten, A. Giuffra, E. Amarger, V. Chardon, P. Andersson-Euklund, L. Andersson, K. Hannsson, I. Lundstrom, K. and Andersson, L. 1999. A paternally expressed QTL affecting skeletal and cardiac muscle mass in pigs maps to the IGF2 locus. Nat. Genetics. 21: 157-158.
Kalscheuer, V.M., Mariman, E.C. Schepens, M.T. Rehder, H. and Ropers, H.H. 1993. The insulin-like growth factor type-2 receptor gene is imprinted in the mouse but not in humans. Nat. Genetics. 5: 74-78.
McLaren, R.J. and Montgomery, G.W. 1999. Genomic imprinting of the insulin-like growth factors 2 gene in sheep. Mamm. Genome 10: 588-591.
Nezer, C., Moreau, L. Brouwers, B. Coppieters, W. Detilleux, J. Hanset, R. Karim, L. Kvasz, A. Leroy, P. and Georges, M. 1999. An imprinted QTL with major effect on muscle mass and fat deposition maps to the IGF2 locus in pigs. Nat. Genetics. 21: 155-156.
Ohlsson, R., Nystrom, A. Pfeifer-Ohlsson, S. Tohonen, V. Hedborg, F. Schofield, P. Flam, F. and Ekstrom, T.J. 1993. IGF2 is parentally imprinted during human embryogenesis and in the Beckwith-Wiedemann syndrome. Nat. Genetics. 4: 94-97.
Ohlsen S.M., Lugenbeel K.A., and E.A. Wong. 1994. Characterization of the Linked Insulin and IGF2 genes. DNA and Cell Bio. 13:377-88.
De Pagter-Holthuizen P., Jansen M., Van Schaik F.M.A., Van der Kammen R., Oosterwijk C., Van de Brande J.L., and J.S. Sussenbach. 1987. The human IGF2 gene contains two development-specific promoters. FEBS Lett. 214:259-64.
De Pagter-Holthuizen P., Jansen M., Van der Kammen R.A., Van Schaik F.M.A, and J.S. Sussenbach. 1988. Differential expression of the human IGF2 gene. Characterization of the IGF2 mRNAs and an mRNA encoding a putative IGF2 associated protein. Biochim.Biophys. Acta 950:282-95.
Rotwein, P. and Hall, L.J. 1990. Evolution of insulin-like growth factor 2: Characterization of the mouse IGF2 gene and identification of two pseudo-exons. DNA. Cell Biol. 9: 725-735.
Sasaki, H., Jones, P.A. Chaillet, J.R. Ferguson-Smith, A.C. Barton, S.C. Reik, W. and Surani, M.A. 1992. Parental imprinting: potentially active chromatin of the repressed maternal allele of the mouse insulin-like growth factor II gene. Genes and Development. 6: 1848-1856.
Schmutz S.M., Moker, J.S. Gallager, Jr.D.S. Kappers, S.M. and Womack, J.E. 1996. In situ hybridization mapping of LDHA and IGF2 to cattle chromosome 29. Mamm. Genome. 7:473.

### SUMMARY OF THE INVENTION:

Recent studies have revealed that a single nucleotide polymorphism in exon 2 of *IGF2* (SNP-2) in cattle is associated with decreased birth weight and increased rib eye area (REA) size. Genomic imprinting in *IGF2* results in expression of only the paternal allele during gestation, while both the maternal and paternal alleles are expressed after birth. The results of these studies also show that the SNP-2 allele from the sire affects birth weight, while both the maternal and paternal IGF2 alleles affect REA size.

With this discovery it will now be possible to use genetic testing to determine an animal's *IGF2* genotype, and then use this knowledge to manage breeding and management programs in order to produce animals that will yield high value meat cuts from the finished animal.

It is well known that genetic variants have the potential to dramatically change the expression and or function of a gene product. In addition single nucleotide polymorphisms (SNPs), whether or not they are the proximate cause of the altered expression or function of the gene product, can further provide a genetic marker for the variant. Thus, where the SNP can associated with a specific phenotype, the SNP provides a further advantage in predicting the phenotypic outcomes based on an animal's genotypic makeup.

The present invention relates to the identification of a single nucleotide polymorphism in exon 2 of *IGF2* (SNP-2) in cattle and to methods of selecting and managing cattle production based on the presence or absence of the polymorphism. The polymorphism comprises a C to T transition at position 150 of SEQ ID NO: 1.

By identifying animals with a particular genotype, with respect to the SNP alleles described herein, it is possible to identify animals that will display the phenotype of a superior carcass quality as compared to animals lacking the desired genotype.

In particular, the present invention relates to methods for establishing the genetically determined predispositions of individual cattle within a group of such animals, to meet particular desired characteristics with respect to growth, based on the association of the specific *IGF2* gene SNPs with observable effects on growth phenotype in animals.

Beever et al. (1990) discloses larger rib eye areas in Angus cattle carrying the chromosomal segment marked by BOLA-w2 allele

It is an object of the invention to predict the quality of a beef carcass, specifically an increase in REA size, through knowledge of the individual animal's SNP-2 genotype.

It is a further object of the present invention to use the *IGF2* exon 2 SNP (SNP-2) as a predictor of *IGF2* expression patterns which are known to result in an increased REA, and to use the knowledge of *IGF2* expression and genomic imprinting in breeding and herd management applications.

The observation that SNP-2 is correlated with increased REA size has implications for both breeding and management practices throughout the various segments of the beef chain or cattle industry. Depending on which part of the chain the producer/company is involved, different management or breeding strategies will be most advantageous in view of an animal's SNP-2 genotype.

The presence of a C residue at position 150 is termed herein as a C allele while the presence of a T residue at position 150 is termed herein as a T allele. Animals can be homozygous with respect to the C allele (C/C cattle) or the T allele (T/T cattle) or heterozygous (C/T cattle).

The SNP-2 genotype affects REA size, such that animals with a C residue at the SNP-2 site in both *IGF2* alleles (C/C offspring) will display the greatest increase in REA size. This invention therefore further allows producers to make breeding/culling decisions based on the knowledge of SNP-2 genotype in the sire and dam in order to obtain C/C offspring, thus maximizing the potential for high value cuts of meat based on predicted REA size.

The present invention is also an improvement on the current method of sorting cattle according to parental phenotypes. As shown in the experimental data that follows, REA size is shown to be predicted by SNP-2 genotype. By enabling a producer to intelligently modulate nutrition provided to animals during phase 1 and 2 of their growth, based on their genetic potential, the economic return on the genetic potential will be maximized.

Animals possessing various *IGF2* SNP genotypes are expected to have different protein and energy requirements. Specifically, animals expressing the C/C genotype, which is associated with increased REA size, can be expected to have higher protein requirements. Present feeding practices, where animals are not distinguished on the invention described herein, may unknowingly under-supply protein to C/C animals and over-supply protein to T/T animals. Using the method of the present invention therefore, producers will be able to maximize the genetic potential of individual animals.

The present invention will also be useful in the management strategies employed in the cattle industry. In the first two phases of the animal's life, which include skeletal and muscular development up to physiological maturity, the nutritional requirements, i.e. protein requirements needed to meet genetic potential for protein accretion, are traditionally based on a group basis, and not on knowledge of individual growth or protein production potential. Thus variation in animal potential is muted by population averaging.

Current methods of sorting use anecdotal frame measurements, classifying animals as small, medium, or large-framed. Once classed in this way, the genetic potential for all the animals in a pen or group are treated as the same. The present invention shows this method of sorting is less precise than sorting by genotype. Using the method of the present invention, sorting animals by their SNP-2 genotype will allow the producer to group cattle within a certain skeletal and muscular range by knowledge of their genotype. This then further allows environmental and nutritional parameters to be tailored to take full advantage of each individual animal's genetic potential for muscular and REA size development which will ultimately increase production efficiencies.

Finally, the method of the present invention will allow those in the slaughter industry to better satisfy their requirements for end beef products, and to more accurately price the animals they purchase for processing. At present REA size is a predictor of high end cuts on the carcass. The greater the REA size, the more valuable the animal. Through application of the method of the present invention, slaughterhouses will know what "value" to expect of a carcass upon processing, based on the knowledge of its SNP-2 genotype. The processing industry will therefore be able to bid for animals more accurately than is currently possible using prior art methods of sorting based on frame size, as they will have knowledge of whether a carcass has a greater or lesser potential to yield higher value cuts of meat.

### DESCRIPTION OF THE DRAWINGS:

While the invention is claimed in the preceding portions hereof, preferred embodiments are provided in the accompanying detailed description which may be best understood in conjunction with the accompanying diagrams, where like parts in each diagram are labeled with like numbers and where:
**Fig. 1****:** A comparison of the genomic organization of the pig, sheep, human, rat, mouse, and cow *IGF2* genes (Adapted from Ohlsen et al. 1994, Amarger et al. 2002). The numbered boxes represent the *IGF2* exons. Open and solid boxes indicate untranslated and translated exons respectively. P1 and P2 are the two pseudo-exons identified in the mouse *IGF2* gene (Rotwein and Hall 1990). Promoters are indicated by P1-P4.

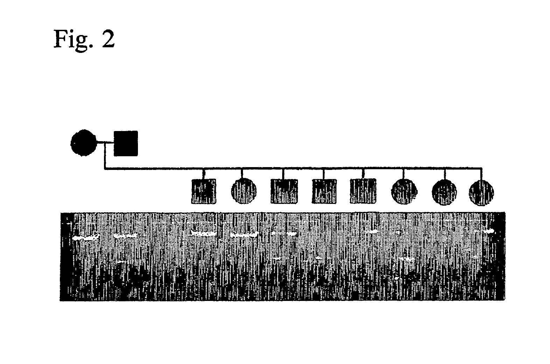

**Fig. 2****:** Photograph of an ethidium bromide stained agarose gel (3%) of an embryo transfer family from a heterozygous (C/T) Charolais sire and homozygous (C/C) Limousin dam. Four calves are heterozygous for the T allele and the other four calves are homozygous for the C allele. The C allele is 185 bp and the T allele is 118 and 68 bp. The 32 bp product is not shown.

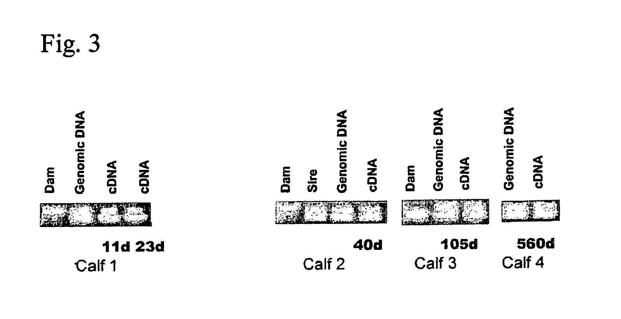

**Fig. 3****:** Photograph of ethidium bromide stained gels from quantitative PCR experiments. The maternal allele is expressed at lower levels that the paternal allele in a young calf (Calf 1). By 40 days of age, there is a complete loss of imprinting in the maternal allele.

### DETAILED DESCRIPTION OF THE INVENTION:

### Definitions:

In the description that follows, a number of terms used in recombinant DNA technology are extensively utilized. In order to provide a clear and consistent understanding of the specification and claims, including the scope to be given such terms, the following definitions are provided:

By "amplifying a segment" as used herein, is meant the production of sufficient multiple copies of the DNA segment to permit relatively facile manipulation of the segment. Manipulation refers to both physical and chemical manipulation, that is, the ability to move bulk quantities of the segment around and to conduct chemical reactions with the segment that result in detectable products.

A "segment" of a polynucleotide refers to an oligonucleotide that is a partial sequence of entire nucleotide sequence of the polynucleotide.

A "modified segment" refers to a segment in which one or more natural nucleotides have been replaced with one or more modified nucleotides. A "modified, labeled segment" refers to a modified segment that also contains a nucleotide, which is different from the modified nucleotide or nucleotides therein, and which is detectably labeled.

An "amplification primer" is an oligonucleotide that is capable of annealing adjacent to a target sequence and serving as an initiation point for DNA synthesis when placed under conditions in which synthesis of a primer extension product which is complementary to a nucleic acid strand is initiated.

By "analysis" is meant either detection of variations in the nucleotide sequence among two or more related polynucleotides or, in the alternative, determining the full nucleotide sequence of a polynucleotide. By "analyzing" the hybridized fragments for an incorporated detectable label identifying the suspected polymorphism is meant that, at some stage of the sequence of events that leads to hybridized fragments, a label is incorporated. The label may be incorporated at virtually any stage of the sequence of events including the amplification, cleavage or hybridization procedures. The label may further be introduced into the sequence of events after cleavage and before or after hybridization. The label so incorporated is then observed visually or by instrumental means. The presence of the label identifies the polymorphism due to the fact that the fragments obtained during cleavage are specific to the modified nucleotide(s) used in the amplification and at least one of the modified nucleotide is selected so as to replace a nucleotide involved in the polymorphism.

The term "animal" is used herein to include all vertebrate animals, including humans. It also includes an individual animal in all stages of development, including embryonic and fetal stages. As used herein, the term "production animals" is used interchangeably with "livestock animals" and refers generally to animals raised primarily for food. For example, such animals include, but are not limited to, cattle (bovine), sheep (ovine), pigs (porcine or swine), poultry (avian), and the like. As used herein, the term "cow" or "cattle" is used generally to refer to an animal of bovine origin of any age. Interchangeable terms include "bovine", "calf", "steer", "bull", "heifer" and the like. As used herein, the term "pig" or is used generally to refer to an animal of porcine origin of any age. Interchangeable terms include "piglet", "sow" and the like.

The term "antisense" is intended to refer to polynucleotide molecules complementary to a portion of an RNA marker of a gene, as defined herein. "Complementary" polynucleotides are those that are capable of base pairing according to the standard Watson-Crick complementarity rules, where purines base pair with pyrimidines to form combinations of guanine paired with cytosine (G:C) and adenine paired with either thymine (A:T) in the case of DNA, or adenine paired with uracil (A:U) in the case of RNA. Inclusion of less common bases like inosine, 5-methylcytosine, 6-methyladenine, hypoxanthine and others in hybridizing sequences does not interfere with pairing.

By the term "complementarity" or "complementary" is meant, for the purposes of the specification or claims, a sufficient number in the oligonucleotide of complementary base pairs in its sequence to interact specifically (hybridize) with the target nucleic acid sequence of the gene polymorphism to be amplified or detected. As known to those skilled in the art, a very high degree of complementarity is needed for specificity and sensitivity involving hybridization, although it need not be 100%. Thus, for example, an oligonucleotide that is identical in nucleotide sequence to an oligonucleotide disclosed herein, except for one base change or substitution, may function equivalently to the disclosed oligonucleotides. A "complementary DNA" or "cDNA" gene includes recombinant genes synthesized by reverse transcription of messenger RNA ("mRNA").

By the term "composition" is meant, for the purposes of the specification or claims, a combination of elements which may include one or more of the following: the reaction buffer for the respective method of enzymatic amplification, plus one or more oligonucleotides specific for *IGF2* gene polymorphisms, wherein said oligonucleotide is labeled with a detectable moiety.

A "cyclic polymerase-mediated reaction" refers to a biochemical reaction in which a template molecule or a population of template molecules is periodically and repeatedly copied to create a complementary template molecule or complementary template molecules, thereby increasing the number of the template molecules over time. The products of such a reaction are commonly referred to as amplification products.

"Denaturation" of a template molecule refers to the unfolding or other alteration of the structure of a template so as to make the template accessible for duplication or hybridization. In the case of DNA, "denaturation" refers to the separation of the two complementary strands of the double helix, thereby creating two complementary, single stranded template molecules. "Denaturation" can be accomplished in any of a variety of ways well known to those skilled in the art, including heat or by treatment of the DNA with a base or other chemical denaturant.

A "detectable amount of product" refers to an amount of amplified nucleic acid that can be detected using standard laboratory tools. A "detectable marker" refers to a nucleotide analog that allows detection using visual or other means. For example, fluorescently labeled nucleotides can be incorporated into a nucleic acid during one or more steps of a cyclic polymerase-mediated reaction, thereby allowing the detection of the product of the reaction using, *e.g.* fluorescence microscopy or other fluorescence-detection instrumentation.

By the term "detectable moiety" is meant, for the purposes of the specification or claims, a label molecule (isotopic or non-isotopic) which is incorporated indirectly or directly into an oligonucleotide, wherein the label molecule facilitates the detection of the oligonucleotide in which it is incorporated when the oligonucleotide is hybridized to amplified gene polymorphism sequences. Thus, "detectable moiety" is used synonymously with "label molecule". Synthesis of oligonucleotides can be accomplished by any one of several methods known to those skilled in the art. Label molecules, known to those skilled in the art as being useful for detection, include chemiluminescent or fluorescent molecules. Various fluorescent molecules are known in the art that are suitable for use to label a nucleic acid substrate for the method of the present invention. The protocol for such incorporation may vary depending upon the fluorescent molecule used. Such protocols are known in the art for the respective fluorescent molecule.

By "detectably labeled" is meant that a fragment or an oligonucleotide contains a nucleotide that is radioactive, that is substituted with a fluorophore or some other molecular species that elicits a physical or chemical response can be observed by the naked eye or by means of instrumentation such as, without limitation, scintillation counters, colorimeters, UV spectrophotometers and the like. As used herein, a "label" or "tag" refers to a molecule that, when appended by, for example, without limitation, covalent bonding or hybridization, to another molecule, for example, also without limitation, a polynucleotide or polynucleotide fragment provides or enhances a means of detecting the other molecule. A fluorescence or fluorescent label or tag emits detectable light at a particular wavelength when excited at a different wavelength. A radiolabel or radioactive tag emits radioactive particles detectable with an instrument such as, without limitation, a scintillation counter. Other signal generation detection methods include: chemiluminescence, electrochemiluminescence, ramanspectroscopy, colorimetric, hybridization protection assay, and Mass spectrometry. "DNA amplification" as used herein refers to any process that increases the number of copies of a specific DNA sequence by enzymatically amplifying the nucleic acid sequence. A variety of processes are known. One of the most commonly used is the polymerase chain reaction (PCR) process of Mullis as described in U.S. Pat. Nos. 4,683,195 and 4,683,202. PCR involves the use of a thermostable DNA polymerase, known sequences as primers, and heating cycles, which separate the replicating deoxyribonucleic acid (DNA), strands and exponentially amplify a gene of interest. Any type of PCR, such as quantitative PCR, RT-PCR, hot start PCR, LA-PCR, multiplex PCR, touchdown PCR, *etc.*, may be used. Preferably, real-time PCR is used. In general, the PCR amplification process involves an enzymatic chain reaction for preparing exponential quantities of a specific nucleic acid sequence. It requires a small amount of a sequence to initiate the chain reaction and oligonucleotide primers that will hybridize to the sequence. In PCR the primers are annealed to denatured nucleic acid followed by extension with an inducing agent (enzyme) and nucleotides. This results in newly synthesized extension products. Since these newly synthesized products become templates for the primers, repeated cycles of denaturing, primer annealing, and extension results in exponential accumulation of the specific sequence being amplified. The extension product of the chain reaction will be a discrete nucleic acid duplex with a termini corresponding to the ends of the specific primers employed.

"DNA" refers to the polymeric form of deoxyribonucleotides (adenine, guanine, thymine, or cytosine) in its either single stranded form, or a double-stranded helix.

This term refers only to the primary and secondary structure of the molecule, and does not limit it to any particular tertiary forms. Thus, this term includes double-stranded DNA found in linear and circular DNA molecules including, but not limited to, restriction fragments, viruses, plasmids, cosmids, and artificial and naturally occurring chromosomes. In discussing the structure of particular double-stranded DNA molecules, sequences may be described herein according to the normal convention of giving only the sequence in the 5' to 3' direction along the non-transcribed strand of DNA (*i.e.*, the strand having a sequence homologous to the mRNA).

By the terms "enzymatically amplify" or "amplify" is meant, for the purposes of the specification or claims, DNA amplification by any process by which nucleic acid sequences are amplified in number. There are several means for enzymatically amplifying nucleic acid sequences known in the art. Currently the most commonly used method is the polymerase chain reaction (PCR). Other amplification methods include LCR (ligase chain reaction) which utilizes DNA ligase, and a probe consisting of two halves of a DNA segment that is complementary to the sequence of the DNA to be amplified, enzyme Qβ replicase and a ribonucleic acid (RNA) sequence template attached to a probe complementary to the DNA to be copied which is used to make a DNA template for exponential production of complementary RNA; strand displacement amplification (SDA); Qβ replicase amplification (QβRA); self-sustained replication (3SR); and NASBA (nucleic acid sequence-based amplification), which can be performed on RNA or DNA as the nucleic acid sequence to be amplified. The particular methodology used to amplify DNA sequences is not intended to be limiting, and all such it is intended that the scope of the invention will include all methods of DNA amplification known in the art.

The "extension of the primers" refers to the addition of nucleotides to a primer molecule so as to synthesize a nucleic acid complementary to a template molecule. "Extension of the primers" does not necessarily imply that the primer molecule is extended to synthesize a complete complementary template molecule. Even if only a fraction of the template molecule has been copied the primer is still considered extended.

A "fragment" of a molecule such as a protein or nucleic acid is meant to refer to any portion of the amino acid or nucleotide genetic sequence.

By "heterozygous" or "heterozygous polymorphism" is meant that the two alleles of a diploid cell or organism at a given locus are different, that is, that they have a different nucleotide exchanged for the same nucleotide at the same place in their sequences.

By "homozygous" is meant that the two alleles of a diploid cell or organism at a given locus are identical, that is, that they have the same nucleotide for nucleotide exchange at the same place in their sequences.

By "hybridization" or "hybridizing," as used herein, is meant the formation of A-T and C-G base pairs between the nucleotide sequence of a fragment of a segment of a polynucleotide and a complementary nucleotide sequence of an oligonucleotide. By complementary is meant that at the locus of each A, C, G or T (or U in the case of an RNA molecule) in the fragment sequence, the oligonucleotide sequenced has a T, G, C or A, respectively. The hybridized fragment/oligonucleotide is called a "duplex." In the case of a DNA-RNA hybrid, the molecular is called a "heteroduplex."

A "hybridization complex", means a complex of nucleic acid molecules including at least the target nucleic acid and sensor probe. It may also include an anchor probe.

By "immobilized on a solid support" is meant that a fragment, primer or oligonucleotide is attached to a substance at a particular location in such a manner that the system containing the immobilized fragment, primer or oligonucleotide may be subjected to washing or other physical or chemical manipulation without being dislodged from that location. A number of solid supports and means of immobilizing nucleotide-containing molecules to them are known in the art; any of these supports and means may be used in the methods of this invention.

As used herein, the term "nucleic acid molecule" is intended to include DNA molecules (*e.g.*, cDNA or genomic DNA), RNA molecules (*e.g.*, mRNA), analogs of the DNA or RNA generated using nucleotide analogs, and derivatives, fragments and homologs thereof. The nucleic acid molecule can be single-stranded or double-stranded, but preferably is double-stranded DNA. An "isolated" nucleic acid molecule is one that is separated from other nucleic acid molecules that are present in the natural source of the nucleic acid. A "nucleoside" refers to a base linked to a sugar. The base may be adenine (A), guanine (G) (or its substitute, inosine (I)), cytosine (C), or thymine (T) (or its substitute, uracil (U)). The sugar may be ribose (the sugar of a natural nucleotide in RNA) or 2-deoxyribose (the sugar of a natural nucleotide in DNA). A "nucleotide" refers to a nucleoside linked to a single phosphate group.

As used herein, the term "oligonucleotide" refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides may be chemically synthesized and may be used as primers or probes. Oligonucleotide means any nucleotide of more than 3 bases in length used to facilitate detection or identification of a target nucleic acid, including probes and primers.

"Polymerase chain reaction" or "PCR" refers to a thermocyclic, polymerase-mediated, DNA amplification reaction. A PCR typically includes template molecules, oligonucleotide primers complementary to each strand of the template molecules, a thermostable DNA polymerase, and deoxyribonucleotides, and involves three distinct processes that are multiply repeated to effect the amplification of the original nucleic acid. The three processes (denaturation, hybridization, and primer extension) are often performed at distinct temperatures, and in distinct temporal steps. In many embodiments, however, the hybridization and primer extension processes can be performed concurrently. The nucleotide sample to be analyzed may be PCR amplification products provided using the rapid cycling techniques described in U.S. Pat. No. 5,455,175. Other methods of amplification include, without limitation, NASBR, SDA, 3SR, TSA and rolling circle replication. It is understood that, in any method for producing a polynucleotide containing given modified nucleotides, one or several polymerases or amplification methods may be used. The selection of optimal polymerization conditions depends on the application.

A "polymerase" is an enzyme that catalyzes the sequential addition of monomeric units to a polymeric chain, or links two or more monomeric units to initiate a polymeric chain. In preferred embodiments of this invention, the "polymerase" will work by adding monomeric units whose identity is determined by and which is complementary to a template molecule of a specific sequence. For example, DNA polymerases such as DNA Pol I and Taq polymerase add deoxyribonucleotides to the 3' end of a polynucleotide chain in a template-dependent manner, thereby synthesizing a nucleic acid that is complementary to the template molecule. Polymerases may be used either to extend a primer once or repetitively or to amplify a polynucleotide by repetitive priming of two complementary strands using two primers.

A "polynucleotide" refers to a linear chain of nucleotides connected by a phosphodiester linkage between the 3'-hydroxyl group of one nucleoside and the 5'-hydroxyl group of a second nucleoside, which in turn is linked through its 3'-hydroxyl group to the 5'-hydroxyl group of a third nucleoside and so on to form a polymer comprised of nucleosides liked by a phosphodiester backbone. A "modified polynucleotide" refers to a polynucleotide in which one or more natural nucleotides have been partially or substantially completely replaced with modified nucleotides.

A "primer" is a short oligonucleotide, the sequence of which is complementary to a segment of the template which is being replicated, and which the polymerase uses as the starting point for the replication process. By "complementary" is meant that the nucleotide sequence of a primer is such that the primer can form a stable hydrogen bond complex with the template; *i.e.*, the primer can hybridize to the template by virtue of the formation of base-pairs over a length of at least ten consecutive base pairs.

The primers herein are selected to be "substantially" complementary to different strands of a particular target DNA sequence. This means that the primers must be sufficiently complementary to hybridize with their respective strands. Therefore, the primer sequence need not reflect the exact sequence of the template. For example, a non-complementary nucleotide fragment may be attached to the 5' end of the primer, with the remainder of the primer sequence being complementary to the strand. Alternatively, non-complementary bases or longer sequences can be interspersed into the primer, provided that the primer sequence has sufficient complementarity with the sequence of the strand to hybridize therewith and thereby form the template for the synthesis of the extension product.

A "restriction enzyme" refers to an endonuclease (an enzyme that cleaves phosphodiester bonds within a polynucleotide chain) that cleaves DNA in response to a recognition site on the DNA. The recognition site (restriction site) consists of a specific sequence of nucleotides typically about 4 - 8 nucleotides long.

A "single nucleotide polymorphism" or "SNP" refers to polynucleotide that differs from another polynucleotide by a single nucleotide exchange. For example, without limitation, exchanging one A for one C, G or T in the entire sequence of polynucleotide constitutes a SNP. Of course, it is possible to have more than one SNP in a particular polynucleotide. For example, at one locus in a polynucleotide, a C may be exchanged for a T, at another locus a G may be exchanged for an A and so on. When referring to SNPs, the polynucleotide is most often DNA and the SNP is one that usually results in a change in the genotype that is associated with a corresponding change in phenotype of the organism in which the SNP occurs.

As used herein, a "template" refers to a target polynucleotide strand, for example, without limitation, an unmodified naturally-occurring DNA strand, which a polymerase uses as a means of recognizing which nucleotide it should next incorporate into a growing strand to polymerize the complement of the naturally-occurring strand. Such DNA strand may be single-stranded or it may be part of a double-stranded DNA template. In applications of the present invention requiring repeated cycles of polymerization, *e.g.*, the polymerase chain reaction (PCR), the template strand itself may become modified by incorporation of modified nucleotides, yet still serve as a template for a polymerase to synthesize additional polynucleotides.

A "thermocyclic reaction" is a multi-step reaction wherein at least two steps are accomplished by changing the temperature of the reaction.

A "thermostable polymerase" refers to a DNA or RNA polymerase enzyme that can withstand extremely high temperatures, such as those approaching 100°C.

Thermostable polymerases are typically isolated from organisms that live in extreme temperatures, such as *Thermus aquaticus*. Examples of thermostable polymerases include Taq, Tth, Pfu, Vent, deep vent, UlTma, and variations and derivatives thereof.

A "variant" is a difference in the nucleotide sequence among related polynucleotides. The difference may be the deletion of one or more nucleotides from the sequence of one polynucleotide compared to the sequence of a related polynucleotide, the addition of one or more nucleotides or the substitution of one nucleotide for another. The terms "mutation," "polymorphism" and "variant" are used interchangeably herein to describe such variants. As used herein, the term "variant" in the singular is to be construed to include multiple variances; *i.e.*, two or more nucleotide additions, deletions and/or substitutions in the same polynucleotide. A "point mutation" refers to a single substitution of one nucleotide for another.

The present invention also makes use of a number of oligonucleotide sequences as described herein as primers for use in DNA amplification reaction or as hybridization probes. It is well known to those skilled in the art that such oligonucleotides may be modified in terms of length and even specificity, and they will still be well suited to the practice of the invention. As such, the invention is not limited to the precise oligonucleotides described but is intended to include all those oligonucleotides that will allow the method of the invention to be carried out as disclosed herein.

### Experimental Examples:

### Materials and Methods:

### Animals

Several sire-dam-calf Holstein families were used to study genomic imprinting.-The 17 full-sib families of the Canadian Beef Reference Herd were used to study the effects of *IGF2* mutations on genomic imprinting.

### PCR

Primers for exon 2 were designed based *Bos taurus* sequence from a cross-bred steer and obtained in our laboratory (GenBank #AY237543).

### SNPs

*Polymerase chain reaction conditions*: PCR reactions of 15 µl contained 1 µl of DNA template (50-100 ng), 1.5 µl of 10X PCR buffer (Invitrogen), 0.45 µl of 50 mM MgCl₂, 0.3 µl of 10 mM dNTPs, 0.1 µl *Taq* polymerase (5 U µl⁻¹: Invitrogen), 1 µl of each primer (10 pM µl⁻¹) and 9.6 µl of ddH₂O). The reaction began with a 4 min step at 94°C, followed by 34 cycles of 50 seconds at 94°C, 50 seconds at 64°C, and 50 seconds at 72°C. Digestion of 8 µl of each PCR product was performed with 1 µl of *Bsr*I (5 U µl-1) for 3 hours at 65°C.
*Primer sequences*:
Forward: 5'- CCTCAGCCTCATCCCCTCCTTTGC - 3' (SEQ ID NO 2)
Reverse: 5'- CTGTGCTCTATTTGCTGTGTTGTCT - 3' (SEQ ID NO 3)

### Results and Discussion:

### Sequencing

Genomic sequence was obtained using the Canadian Beef Reference Herd of 17 full-sub cattle families, from 5 sires of the CBRH families and two Holstein calves, as well as the original crossbred steer. A portion of the sequence of the IGF2 gene is shown as SEQ ID NO: 1. Two, single nucleotide polymorphisms (SNPs) were discovered in the *IGF2* gene. One SNP was located in the 3' end of intron 8, and a second in the middle portion of exon 2 (SNP-2).

A 217 bp PCR fragment of the *IGF2* gene (SEQ ID NO: 1) was found to contain an internal *Bsr*I restriction site at position 32. The presence of SNP-2 (a C - T transition) at nucleotide 150 resulted in the creation of an additional *Bsr*I restriction site. Thus, after digestion with *Bsr*I DNA derived from the C allele yielded *Bsr*I fragments of 32 and 185 bp, while DNA derived from the T allele yielded *Bsr*I fragments of 32, 67 and 118 bp fragments. Alleles were resolved on a 3% agarose gel (Figs. 2).

### Genomic Imprinting

SNP-2 was used to determine if *IGF2* was paternally expressed in cattle. To test for genomic imprinting, tissues were collected from several Holstein calves heterozygous for SNP-2. The calves ranged from 1 to 560 days of age. The mother and father of the calf were also genotyped to assign a parent of origin to each allele in an informative family. Messenger RNA was extracted and analyzed for parental allele expression. At 1 day of age calves expressed only the paternal allele. By 11 days of age expression IGF2 mRNA from the maternal allele was detectable, but was at levels less than those observed for the paternal allele. However, by 4 weeks of age, all calves expressed mRNA from both maternal and paternal alleles equally (Fig. 3).

A loss of imprinting of *IGF2* has been found in humans and sheep as the animal ages. In sheep, by 6 months of age the liver had lost its imprint and *IGF2* was expressed similarly from both the maternal and paternal alleles. In contrast, expression in the kidney still displayed the effects of imprinting (McLaren and Montgomery 1999). Such a loss of imprinting has not been demonstrated in rodents, probably due to the lack of a functional equivalent to the human P1 promoter (Ohlsen et al. 1994).

### Effects

Since in most of the tissues examined exon 2 is not transcribed, SNP-2 would not be expected to have any direct effect on either the structure or levels of the IGF2 protein. However, in liver, exon 2 is transcribed. Thus, mutations in exon 2 could affect *IGF2* gene expression, either through changes in stability or translational efficiency of the *IGF2* mRNA, even though the exon 2 sequences does not encode part of the final IGF2 protein. SNP-2 might also causes changes in transcriptional efficiency.

Preliminary analysis examining the effects of SNP-2 suggested a correlation with increased rib eye area (REA) size. REA size was corrected for sex of offspring; 108 of the offspring were either C/C or C/T. In animal 19 - 20 months old, heterozygous C/T offspring had a significantly smaller REA size than homozygous C/C offspring (t = 3.625, P = 0.0004). The mean REA in C/C animals was 109.8 cm² (n = 93) as compared to 100.4 cm² (n = 29) in the C/T animals. In the 125 cattle studied, REA size was significantly correlated with the number of C alleles present in the population (r = 0.39, P =0.0001).

The analysis of a second data set provided further support for a correlation between SNP-2 and increased REA. In the second study of 167 bulls, REA was positively correlated with the number of C alleles in the population (r = 0.155, P = 0.0459). Cattle with a C/C genotype had a significantly larger REA than the C/T or T/T genotype cattle (t=2.057, P = 0.0413). Although REA size was also correlated with carcass weight (r = 0.382, P = 0.0001), there was no significant correlation between SNP-2 and sex-corrected carcass weight (r = 0.096, P = 0.2855). This suggests that *IGF2* affects the muscle growth but not overall growth (total animal carcass weight).

When the effect of SNP-2 on REA size and fat was examined, it was also discovered that the C allele is associated with increased REA size and decreased fat while the T allele is associated with decreased REA size and increased fat. The net effect is that there not effect on the overall carcass weight. Consistent with this interpretation, further studies revealed that SNP-2 had a significant effect on sex corrected fat (t = - 1.881, P = 0.0624) and sex corrected marbling (t = -1.874, P = 0.0633) in the C/C cattle. The C/C animals had a mean fat of 9.07 and a mean marbling of 457.7 (n = 93) while the C/T animals had a mean fat of 10.68 and a mean marbling of 485.0 (n = 29). These results indicate that *IGF2* affects fat and may also function as a partitioning agent. These results further demonstrate that SNP-2 affects muscle size but not overall weight. The effect on REA is significantly correlated with the number of C alleles suggesting a co-dominant inheritance pattern.

REA size was correlated to raw birth weight (r = 0.188, P = 0.0001). Preliminary analysis examining the effects of the SNP-2 suggested a correlation with birth weight depending on the allele inherited from the sire. Birth weight was corrected for sex of offspring: 108 animals had a paternal C allele and 14 had a T allele. Offspring having a paternal C allele had a significantly lower birth weight than offspring who had inherited a paternal T allele (t = 2.516, P = 0.0132). The mean sex corrected birth weight for paternal C allele offspring was 112.9 lbs (n = 108) compared to 123.3 lbs (n = 14) in the paternal T offspring. No such effect was observed when the maternal allele was considered (t = 0.992, P = 0.3234).

The result of the present studies is that *IGF2* SNP-2 can be used to predict REA size in cattle offspring based on the knowledge of the animal's genotype. The invention thus provides a more accurate method of predicting REA size, and is an improvement on the prior method of phenotypic sorting of animals based on frame size, which we know is not well correlated with REA size.

In addition, the SNP-2 genotype of the sire can be used to predict birth weight of calves. Since the maternal IGF2 allele is not expressed during gestation, it does not influence birth weight of the calf. Therefore C/C sires that will pass on an *IGF2* C allele to their offspring and thus will produce only "low birth weight" calves can be selected and marketed based on this finding. In contrast, where a producer desires higher birth weight calves, breeding T/T sires will produce calves of this phenotype. Where a mixed herd is desired, breeding of C/T/ sires will results in offspring of a high vs. low birth weight in an approximately equal numbers as a result of normal Mendelian segregation of alleles.

## Claims

1. A method for identifying a phenotype in cattle, the method comprising:
detecting a polymorphism present in the *IGF2* gene at position 150 of SEQ ID NO: 1; and
wherein the presence of a C residue (a C allele) is associated with the phenotype of increased rib eye area, as compared to cattle with a T residue (T allele) at position 150 of SEQ ID NO: 1.

2. The method of claim 1 wherein detecting the polymorphism comprises:
isolating a genomic DNA sample from cattle;
amplifying a region of the bovine *IGF2* gene using an oligonucleotide pair to form nucleic acid amplification products comprising amplified *IGF2* gene polymorphism sequences;
analyzing the amplification products to determine the presence or absence of at least one C allele.

3. The method of claim 2 wherein the oligonucleotide pair comprises SEQ ID NO: 2 and SEQ ID NO: 3.

4. The method of claim 3 wherein the polymorphism detected is a restriction fragment length polymorphism (RFLP).

5. The method of claim 4 wherein the RFLP is the presence or absence of a *BsrI* restriction site at nucleotide 150 in a nucleic acid amplification product produced by amplification of a portion of the *IGF2* gene using the oligonucleotide pair SEQ ID NO: 2 and SEQ ID NO: 3.

6. The method of claim 2 further comprising the inclusion of a detectable moiety such that the amplification product comprises a labeled amplification product.

7. The method of claim 6 wherein the detectable moiety is selected from the group consisting of fluorescent, bioluminescent, chemiluminescent, radioactive and colorigenic moieties.

8. The method of claim 1 further comprising:
contacting the nucleic acid amplification products with a hybridization probe;
wherein the hybridization probes comprise at least one oligonucleotide labeled with a detectable moiety;
under suitable conditions permitting hybridization of the at least one oligonucleotide to the amplification product to form a hybridization complex; and
wherein the presence of the detectable moiety in the hybridization complex indicates the presence of a *IGF2* polymorphism.

9. The method of claim 1 wherein the nucleic acid amplification product is produced by an amplification method selected from the group of polymerase chain reaction (PCR), strand displacement amplification (SDA), nucleic acid sequence based amplification (NASBA), rolling circle amplification, T7 polymerase mediated amplification, T3 polymerase mediated amplification and SP6 polymerase mediated amplification.

10. An isolated and purified nucleic acid comprising a portion of the bovine *IGF2* gene, further comprising a polymorphism at position 150 as defined by the positions in SEQ ID NO: 1, and in which there is a C residue or a T residue at position 150.

11. A method of selecting individual cattle based on the knowledge of an animal's *IGF2* genotype, comprising the steps of:
determining the *IGF2* alleles of an animal;
wherein the alleles of an animal are one of C/C, CT, or T/T with respect to position 150 of SEQ ID NO: 1; and
sorting animals into groups of like genotype; and
wherein a C/C or C/T genotype is associated with the phenotype of increased rib eye area, as compared to T/T cattle.

12. A diagnostic kit for determining the *IGF2* genotype of a bovine animal, the kit comprising:
oligonucleotide primers for amplifying a portion of the *IGF2* gene;
the primers comprising a forward primer comprising, at it's 3' end, sequence identical to at least 10 contiguous nucleotides within SEQ ID NO: 1;
a reverse primer comprising, at it's 3' end, a nucleotide sequence fully complementary to at least 10 contiguous nucleotides with SEQ ID NO: 1;
and wherein the forward and reverse primers will produce, in a PCR amplification reaction, a nucleic acid product amplification product containing a residue corresponding to position 150 of SEQ ID NO: 1.

13. The kit of claim 12 wherein the primers comprise the oligonucleotides SEQ ID NO: 2 and SEQ ID NO: 3.

14. The kit of claim 12 wherein the primers are labeled with a detectable moiety.

15. The kit of claim 12 further comprising at least one oligonucleotide, labeled with a detectable moiety and suitable for use as a hybridization probe.

## Patentansprüche

1. Verfahren zum Identifizieren eines Phänotyps bei Rindern, wobei das Verfahren umfasst:
Erkennen eines Polymorphismus, der in dem *IGF2*-Gen an Position 150 der Seq ID Nr. 1 vorhanden ist; und
wobei die Anwesenheit eines C-Rests (eines C-Allels) mit dem Phänotyp des vergrößerten Hochrippenbereichs assoziiert ist, verglichen mit Rindern mit einem T-Rest (T-Allel) an Position 150 der Seq ID Nr. 1.

2. Verfahren nach Anspruch 1, wobei das Erkennen des Polymorphismus umfasst:
Isolieren einer genomischen DNA-Probe aus Rindern;
Amplifizieren eines Bereichs des Rinder-*IGF2*-Gens unter Verwendung eines Oligonukleotidpaars, um Nukleinsäurenamplifikationsprodukte zu bilden, die amplifizierte *IGF2*-Genpolymoiphismussequenzen aufweisen;
Analysieren der Amplifikationsprodukte, um die Anwesenheit oder Abwesenheit mindestens eines C-Allels zu bestimmen.

3. Verfahren nach Anspruch 2, wobei das Oligonukleotidpaar Seq ID Nr. 2 und Seq ID Nr. 3 umfasst.

4. Verfahren nach Anspruch 3, wobei der erkannte Polymorphismus ein Restriktionsfragmentlängenpolymorphismus (RFLP) ist.

5. Verfahren nach Anspruch 4, wobei der RFLP die Anwesenheit oder Abwesenheit einer *BsrI*-Restriktionsstelle an Nukleotid 150 in einem Nukleinsäurenamplifikationsprodukt ist, das durch Amplifikation eines Abschnitts des *IGF2*-Gens unter Verwendung des Oligonukleotidpaars Seq ID Nr. 2 und Seq ID Nr. 3 hergestellt wird.

6. Verfahren nach Anspruch 2, weiterhin umfassend den Einschluss eines erkennbaren Rests, so dass das Amplifikationsprodukt ein gekennzeichnetes Amplifikationsprodukt umfasst.

7. Verfahren nach Anspruch 6, wobei der erkennbare Rest aus der Gruppe bestehend aus fluoreszierenden, biolumineszierenden, chemilumineszierenden, radioaktiven und kolorigenen Resten ausgewählt ist.

8. Verfahren nach Anspruch 1, weiterhin umfassend:
Kontaktieren der Nukleinsäurenamplifikationsprodukte mit einer Hybridisierungssonde;
wobei die Hybridisierungssonde mindestens ein mit einem erkennbaren Rest gekennzeichnetes Oligonukleotid umfasst;
unter geeigneten Bedingungen das Zulassen der Hybridisierung des mindestens einen Oligonukleotids zu dem Amplifikationsprodukt, um einen Hybridisierungskomplex zu bilden; und
wobei die Anwesenheit des erkennbaren Rests in dem Hybridisierungskomplex die Anwesenheit eines *IGF2*-Poylmorphismus anzeigt.

9. Verfahren nach Anspruch 1, wobei das Nukleinsäurenamplifikationsprodukt durch ein Amplifikationsverfahren erzeugt wird, das aus der Gruppe von Polymerase-Kettenreaktion (PCR), Strangverdrängungsamplifikation (SDA), Nukleinsäurensequenz-basierende Amplifikation (NASBA), Rolling-Circle-Amplifikation, T7-Polymerase-vermittelter Amplifikation, T3-Polymerase-vermittelter Amplifikation und SP6-Polymerase-vermittelter Amplifikation ausgewählt ist.

10. Isolierte und gereinigte Nukleinsäure, umfassend einen Abschnitt des Rinder-*IGF2-*Gens, weiterhin umfassend einen Polymorphismus an Position 150, wie durch die Positionen in Seq ID Nr. 1 definiert, und in der sich an Position 150 ein C-Rest oder ein T-Rest befindet.

11. Verfahren zum Auswählen einzelner Rinder basierend auf der Kenntnis des *IGF2-*Genotyps eines Tiers, umfassend folgende Schritte:
Bestimmen der *IGF2*-Allele eines Tiers;
wobei die Allele eines Tiers eines aus C/C, C/T oder T/T bezogen auf Position 150 der Seq ID Nr. 1 sind; und
Sortieren von Tieren in Gruppen des gleichen Genotyps;
wobei ein C/C- oder C/T-Genotyp, verglichen mit T/T-Rindern, mit dem Phänotyp des vergrößerten Hochrippenbereichs assoziiert ist.

12. Diagnostischer Kit zum Bestimmen des *IGF2*-Genotyps eines Rinds, wobei der Kit Folgendes umfasst:
Oligonukleotidprimer zum Amplifizieren eines Abschnitts des *IGF2*-Gens;
wobei die Primer einen Vorwärtsprimer umfassen, der an seinem 3'-Ende eine Sequenz umfasst, die mit mindestens 10 zusammenhängenden Nukleotiden innerhalb von Seq ID Nr. 1 identisch ist,
einen Rückwärtsprimer, der an seinem 3'-Ende eine Nukleotidsequenz umfasst, die zu mindestens 10 zusammenhängenden Nukleotiden in Seq ID Nr. 1 vollständig komplementär ist,
und wobei die Vorwärts- und Rückwärtsprimer in einer PCR-Amplifikationsreaktion ein Nukleinsäureprodukt-Amplifikationsprodukt erzeugen, das einen Rest enthält, der mit Position 150 von Seq ID Nr. 1 korrespondiert.

13. Kit nach Anspruch 12, wobei die Primer die Oligonukleotide Seq ID Nr. 2 und Seq ID Nr. 3 umfassen.

14. Kit nach Anspruch 12, wobei die Primer mit einem erkennbaren Rest **gekennzeichnet** sind.

15. Kit nach Anspruch 12, weiterhin umfassend mindestens ein Oligonukleotid, das mit einem erkennbaren Rest **gekennzeichnet** ist und zur Verwendung als Hybridisierungssonde geeignet ist.

## Revendications

1. Procédé pour identifier un phénotype chez le bovin, comprenant :
la détection d'un polymorphisme présent dans le gène *IGF2* en position 150 de la SEQ ID NO : 1 ; et
dans lequel la présence d'un résidu C (un allèle C) est associée au phénotype de muscle longissimus dorsi étendu, par comparaison avec un bovin ayant un résidu T (allèle T) en position 150 de la SEQ ID NO : 1.

2. Procédé selon la revendication 1, dans lequel la détection du polymorphisme comprend :
l'isolement d'un échantillon d'ADN génomique à partir d'un bovin ;
l'amplification d'une région du gène *IGF2* de bovin par utilisation d'une paire d'oligonucléotides pour former des produits d'amplification d'acide nucléique comprenant des séquences de polymorphisme de gène *IGF2* amplifiées ;
l'analyse des produits d'amplification pour déterminer la présence ou l'absence d'au moins un allèle C.

3. Procédé selon la revendication 2, dans lequel la paire d'oligonucléotides comprend la SEQ ID NO : 2 et la SEQ ID NO : 3.

4. Procédé selon la revendication 3, dans lequel le polymorphisme détecté est un polymorphisme de longueur de fragment de restriction (RFLP).

5. Procédé selon la revendication 4, dans lequel le RFLP est la présence ou l'absence d'un site de restriction *BsrI* au nucléotide 150 dans un produit d'amplification d'acide nucléique produit par amplification d'une partie du gène *IGF2* en utilisant la paire de nucléotides SEQ ID NO : 2 et SEQ ID NO : 3.

6. Procédé selon la revendication 2, comprenant en outre l'inclusion d'un fragment détectable de façon que le produit d'amplification comprenne un produit d'amplification marqué.

7. Procédé selon la revendication 6, dans lequel le fragment détectable est choisi dans le groupe constitué par des fragments fluorescents, bioluminescents, chimioluminescents, radioactifs et colorigènes.

8. Procédé selon la revendication 1, comprenant en outre :
la mise en contact des produits d'amplification d'acide nucléique avec une sonde d'hybridation ;
les sondes d'hybridation comprenant au moins un oligonucléotide marqué avec un fragment détectable ;
dans des conditions convenables permettant l'hybridation dudit au moins un oligonucléotide au produit d'amplification pour former un complexe d'hybridation ; et
dans lequel la présence du fragment détectable dans le complexe d'hybridation indique la présence d'un polymorphisme *IGF2*.

9. Procédé selon la revendication 1, dans lequel le produit d'amplification d'acide nucléique est produit par un procédé d'amplification choisi dans le groupe constitué par une amplification en chaîne par polymérase (PCR), une amplification par déplacement de brin (SDA), une amplification basée sur une séquence d'acide nucléique (NASBA), une amplification en cercle roulant, une amplification à médiation par la T7 polymérase, une amplification à médiation par la T3 polymérase et une amplification à médiation par la SP6 polymérase.

10. Acide nucléique isolé et purifié comprenant une partie du gène *IGF2* de bovin, comprenant en outre un polymorphisme en position 150 comme défini par les positions dans la SEQ ID NO : 1, et dans lequel il y a un résidu C ou un résidu T en position 150.

11. Procédé pour sélectionner un bovin individuel sur la base de la connaissance d'un génotype *IGF2* de l'animal, comprenant les étapes consistant à :
déterminer les allèles *IGF2* d'un animal ;
les allèles d'un animal étant l'une parmi C/C, CT et T/T par rapport à la position 150 de la SEQ ID NO : 1 ; et
trier les animaux en groupes de génotype analogue ; et
dans lequel un génotype C/C ou C/T est associé au phénotype de muscle longissimus dorsi étendu, par comparaison avec le bovin T/T.

12. Kit de diagnostic pour déterminer le génotype *IGF2* d'un animal bovin, comprenant :
des amorces oligonucléotidiques pour amplifier une partie du gène *IGF2* ;
les amorces comprenant une amorce sens comprenant, à son extrémité 3', une séquence identique à au moins 10 nucléotides contigus à l'intérieur de la SEQ ID NO : 1 ;
une amorce antisens comprenant, à son extrémité 3', une séquence de nucléotides entièrement complémentaire à au moins 10 nucléotides contigus de la SEQ ID NO : 1 ;
et dans lequel les amorces sens et antisens vont produire, dans une réaction d'amplification PCR, un produit d'amplification de produit d'acide nucléique contenant un résidu correspondant à la position 150 de la SEQ ID NO : 1.

13. Kit selon la revendication 12, dans lequel les amorces comprennent les oligonucléotides SEQ ID NO : 2 et SEQ ID NO : 3.

14. Kit selon la revendication 12, dans lequel les amorces sont marquées avec un fragment détectable.

15. Kit selon la revendication 12, comprenant en outre au moins un oligonucléotide, marqué avec un fragment détectable et utilisable en tant que sonde d'hybridation.


### SEQUENCE LISTING

(1) GENERAL INFORMATION:
   (i) APPLICANT:
      Schmutz, Sheila Marie
      RR # 2, Box 123
      Saskatoon, SK, Canada
      S7K 3J5

      Julie Janine Goodall
      101B-815 Reid Road,
      Saskatoon, SK, Canada
      S7N 2W8
   (ii) TITLE OF INVENTION:
      Improving Production Characteristics of Cattle
   (iii) NUMBER OF SEQUENCES: 3
   (iv) CORRESPONDENCE ADDRESS:
      Furman & Kallio
      1400 - 2002 Victoria Avenue
      Regina, SK, Canada
      S4P 0R7
   (v) COMPUTER-READABLE FORM:
      COMPUTER: Dell Server
      OPERATING SYSTEM: Windows XP Professional
      SOFTWARE: Microsoft Word - Office 2004 Version
   (vi) CURRENT APPLICATION DATA:
      APPLICATION NUMBER:
      FILING DATE:
      CLASSIFICATION:
   (vii) PRIOR APPLICATION DATA:
      APPLICATION NUMBER: US 60/489,026
      FILING DATE: July 23, 2003
      CLASSIFICATION:
   (viii) PATENT AGENT INFORMATION:
      a) NAME:
         Furman & Kallio
         1400 - 2002 Victoria Avenue
         Regina, SK, Canada
         S4P 0R7
      b) REFERENCE NUMBER: 1709-03-00
(2) INFORMATION FOR SEQ ID NO: 1
   (i) SEQUENCE CHARACTERISTICS:
      (a) LENGTH: 217 base pairs
      (b) TYPE: nucleotide
      (c) STRANDEDNESS: double stranded
      (d) TOPOLOGY: linear
   (ii) MOLECULE TYPE: genomic DNA
   (iii) HYPOTHETICAL: no
   (iv) ANTI-SENSE: no
   (vi) ORIGINAL SOURCE: Bos taurus
   (viii) POSITION IN GENOME:
      a) CHROMOSOME SEGMENT: BTA 29
      b) MAP POSITION: 0 cM from ILSTS081
      c) UNITS: centiMorgans
   (ix) FEATURE:
      a) NAME/KEY: SNP-2
      b) LOCATION: bp 150
      c) IDENTIFICATION METHOD: DNA sequencing of PCR products
      d) OTHER INFORMATION: creates an additional BsrI restriction site; affects birth weight, sex-corrected rib eye area size, fat content and marbling
   (x) PUBLICATION INFORMATION:
      a) AUTHORS: Goodall, J.J. and Schmutz, S.M.
      b) TITLE: Linkage Mapping of IGF2 on Cattle Chromosome 29
      c) JOURNAL: Animal Genetics
      d) VOLUME: 34
      e) ISSUE: 4
      f) PAGES: 313 - 313
      j) PUBLICATION DATE: August 2003
      k) RELEVANT RESIDUES IN SEQUENCE ID NO: 2: C or T residue at position 150
   (xi) SEQUENCE DESCRIPTION: SEQ ID NO: 1 
cctcagcctc atcccctcct ttgcctccca gtcaagcctg ctggggggtc tgagcaccac 61

agccagagca cccccgcctt ggcagcgact gctactattg gcccagccag cggatcatcc 121

acctggcagt cgagagcctg gggcaccagy gacgtccagg tcctctttac ccaccgccca 181
 ggggagcttc agagacaaca cagcaaatag agcacag 217
(2) INFORMATION FOR SEQ ID NO: 2
   (i) SEQUENCE CHARACTERISTICS:
      (a) LENGTH: 24 nucleotides
      (b) TYPE: nucleotide
      (c) STRANDEDNESS: single stranded
      (d) TOPOLOGY: linear
   (ii) MOLECULE TYPE: DNA (primer)
   (iii) HYPOTHETICAL: no
   (iv) ANTI-SENSE: no
   (xi) SEQUENCE DESCRIPTION: SEQ ID NO: 2
      cctcagcctc atcccctcct ttgc 24
(2) INFORMATION FOR SEQ ID NO: 3
   (i) SEQUENCE CHARACTERISTICS:
      (a) LENGTH: 25 nucleotides
      (b) TYPE: nucleotide
      (c) STRANDEDNESS: single stranded
      (d) TOPOLOGY: linear
   (ii) MOLECULE TYPE: DNA (primer)
   (iii) HYPOTHETICAL: no
   (iv) ANTI-SENSE: no
   (xi) SEQUENCE DESCRIPTION: SEQ ID NO: 3
      ctgtgctcta tttgctgtgt tgtct 25